# EUROPÄISCHE PATENTSCHRIFT

(11) **EP 2 262 535 B1**
(45) Veröffentlichungstag und Bekanntmachung des Hinweises auf die Patenterteilung: **10.07.2013**
(21) Anmeldenummer: 09732467.7
(22) Anmeldetag: 05.02.2009
(51) Int. Cl.: A61K 31/734, A61K 33/10, A61K 35/02, A61K 45/06, A61P 1/04

(54) **VERWENDUNG EINER HEILERDE-ENTHALTENDEN ZUSAMMENSETZUNG ALS ANTAZIDUM**
USE OF A COMPOSITION CONTAINING HEALING EARTH AS AN ANTACID
UTILISATION D'UNE COMPOSITION CONTENANT DE LA TERRE CURATIVE COMME ANTI-ACIDE

(30) Priorität: 16.04.2008 DE 102008019339
(43) Veröffentlichungstag der Anmeldung: 22.12.2010
(73) Patentinhaber: Maria Clementine Martin Klosterfrau Vertriebsgesellschaft mbH, 50670 Köln (DE)
(72) Erfinder: WESTWEBER, Anna-Maria, 51399 Burscheid (DE); SCHÜLTER, Andreas, 29439 Lüchow (DE); HOFFMANN, Hans-Georg, 50996 Köln (DE)
(74) Vertreter: Von Rohr
(86) Internationale Anmeldenummer: PCT/EP2009/000794
(87) Internationale Veröffentlichungsnummer: WO 2009/127283

(56) Entgegenhaltungen:
- EP-A- 0 506 563
- FR-A- 2 669 822
- US-A1- 2007 281 015
- HOLLRIEGL ET AL: "Observation of changes in urinary excretion of thorium in humans following ingestion of a therapeutic soil" JOURNAL OF ENVIRONMENTAL RADIOACTIVITY, ELSEVIER APPLIED SCIENCE PUBLISHERS, BARKING, GB, Bd. 95, Nr. 2-3, 1. Juni 2007 (2007-06-01), Seiten 149-160, XP022115165 ISSN: 0265-931X
- B. UEHLEKE: "Luvos-Heilerde - ein bewährtes Naturheilmittel im Blick neuer Forschung" NATUR-HEILKUNDE JOURNAL, MEDIZIN PRAXIS WISSENSCHAFT, 1. Oktober 2005 (2005-10-01), XP002541877 Gefunden im Internet: URL:http://www.luvos.de/data/luvos/media/d oc/Naturheilkunde_Journal_10_2005.pdf>

## Beschreibung

Die vorliegende Erfindung betrifft eine Zusammensetzung (d. h. eine pharmazeutische Zusammensetzung bzw. Zubereitung), insbesondere ein Antazidum, vorzugsweise in fester Dosierungsform (z. B. als Pulver, als Granulat oder als Tablette) oder in flüssiger Dosierungsform (z. B. als Suspension), welche - in jeweils wirksamen bzw. pharmazeutisch wirksamen Mengen - eine Kombination von Heilerde einerseits und Alginsäure und/oder Alginsäuresalzen andererseits zusammen mit mindestens einer unter physiologischen Bedingungen im Magen gasfreisetzenden Komponente umfaßt, wobei die Zusammensetzung die in Anspruch 1 angsgeberen Mengen der Bestandteile enthält.

Weiterhin betrifft die vorliegende Erfindung die Verwendung der erfindungsgemäßen Zusammensetzung bzw. der erfindungsgemäßen pharmazeutischen Zusammensetzung, insbesondere des erfindungsgemäßen Antazidums, zur Herstellung eines Anzneimittels zur prophylaktischen und/oder kurativen, insbesondere symptomatischen Behandlung von Hyperazidität des Magens - synonym bisweilen auch als "Hyperchlorhydrie" oder "Magen(saft)übersäuerung/- überproduktion" bezeichnet- und damit in Zusammenhang stehenden Folgeerscheinungen und Folgeerkrankungen, wie beispielsweise Sodbrennen, Ösophagitis (z. B. Refluxösophagitis), dyspeptischen Beschwerden, Reizmagen, Gastritis, Ulcuserkrankungen und dergleichen.

Magensäure ist eine sehr starke Säure. Die Magensäure hat die physiologischen Aufgaben, die Speisen zu verdauen und Krankheitskeime aus der Nahrung unschädlich zu machen. Damit die Magensäure nicht das umliegende Körpergewebe verdaut und schädigt, ist die Magenwand mit einer Schutzschicht ausgekleidet. Die Öffnungen zur Speiseröhre und zum Zwölffingerdarm sind mit Schließmuskeln abgedichtet. Diese Muskeln sorgen dafür, daß der Speisebrei von der Speiseröhre in den Magen und vom Magen in den Dünndarm fließt und nicht umgekehrt.

Die Magensäure wird in der Magenschleimhaut hauptsächlich in der Nacht produziert und dort auch gespeichert. Tagsüber wird sie dann - je nach Bedarf - aus den Speichern in den Magen abgegeben. Die Menge an Säure, die jeweils freigesetzt wird, hängt von vielen Faktoren ab. So wird besonders viel Magensäure bei reichhaltigem bzw. fettem Essen, aber auch durch Streß freigesetzt.

Entscheidend dabei ist aber das sinnvolle physiologische Gleichgewicht zwischen aggressiven und defensiven Vorgängen im Magen; kommt es zu einem Ungleichgewicht, entstehen Magenprobleme von Sodbrennen bis hin zur Gastritis oder zum Magengeschwür.

Normalerweise funktioniert das zuvor geschilderte, ausgeklügelte System einwandfrei. Doch manchmal können Nahrungsmittel, Genußmittel, aber auch Krankheiten und Medikamente Beschwerden in Verbindung mit der Magensäure hervorrufen. Die Beschwerden bei Sodbrennen und Magenbeschwerden sind sehr ähnlich und oft nur schwer zu unterscheiden. Da die Magensäure durch Nahrung gebunden wird und dadurch an Aggressivität verliert, treten die Beschwerden meist erst einige Zeit, üblicherweise etwa eine bis zwei Stunden nach dem Essen auf oder aber nachts.

Eine sogenannte Hyperazidität des Magens kann unterschiedliche Ursachen haben: In vielen Fällen gibt es keine bekannten bzw. ermittelbaren Ursachen für die Neigung zu Sodbrennen und anderen, mit der Hyperazidität in Verbindung stehenden Folgeerscheinungen und Folgeerkrankungen; jedoch kann bisweilen einer der folgenden Gründe ausschlaggebend sein: So können übermäßiger Genuß von Kaffee, Alkohol und Nikotin, üppige, insbesondere fette Mahlzeiten, stark gewürzte und gebratene Speisen, Zitrusfrüchte, Fruchtsäfte, Zwiebeln, Schokolade, Tomaten etc. solche Beschwerden hervorrufen. Auch Streß und falsche Eßgewohnheiten (z. B. hastiges Hinunterschlingen von Speisen) können Ursache für eine Hyperazidität des Magens sein. Übergewichtige und Schwangere leiden häufig unter Sodbrennen, da die Größe und Körpermasse auf den Magen drücken. Auch Verstopfungen, Blähungen, eine erhöhte Magensäureproduktion oder eine verzögerte Magenentleerung können Ursachen für eine Hyperazidität des Magens sein. Auch können manche Medikamente als Nebenwirkung den Schließmuskel zur Speiseröhre erschlaffen lassen. Auch organische Ursachen, wie beispielsweise eine Erschlaffung des Schließmuskels, eine "verrutschte" Magenwand oder eine Hernie, können Ursache für eine Hyperazidität des Magens sein. Auch kann die Langzeiteinnahme von Schmerzmitteln aus der Gruppe der nichtsteroidalen Antiphlogistika, welche die Bildung der schützenden Prostaglandine hemmen, zu einer Hyperazidität des Magens führen.

Zunächst kommt es infolge der Hyperazidität zu Sodbrennen, verbunden mit einem brennenden Schmerz in der Brust. Wenn der Schließmuskel zwischen Magen- und Speiseröhre nicht optimal funktioniert fließt saurer Magenbrei in die Speiseröhre zurück und reizt dort die ungeschützte Schleimhaut, was zu einer sogenannten Refluxösophagitis führen kann. Gelangt der Magensaft sogar in den Mund, spricht man von saurem Aufstoßen. Sodbrennen tritt meist circa eine bis zwei Stunden nach den Mahlzeiten oder beim Bücken und Liegen auf.

Das Sodbrennen selbst ist keine Krankheit, sondern eine Beschwerde, welche mit unterschiedlichen Krankheiten zusammenhängen kann. Gelegentliches Sodbrennen ist unbedenklich. Tritt es jedoch mehrmals pro Woche auf, verätzt die Magensäure auf Dauer die empfindliche Schleimhaut der Speiseröhre. Die Folge kann eine Refluxösophagitis sein. Bei einer chronischen, also immer wiederkehrenden Speiseröhrenentzündung besteht die Gefahr, daß die betroffenen Schleimhautzellen entarten und sich ein Speiseröhrenkarzinom entwikkeln kann.

Eine leichte Reizung der Magenschleimhaut kann gleichermaßen eine Magenschleimhautentzündung (Gastritis) hervorrufen, welche im allgemeinen schnell abklingt. Bei Dauerreizung kann die Entzündung aber auch chronisch werden. Im Laufe der Zeit kann sich daraus eine Wunde in der Schleimhaut, insbesondere ein Magengeschwür, entwickeln. Auch hier besteht die Gefahr, daß die Schleimhautzellen entarten und sich zu einem Karzinom entwickeln.

Bei einem Reizmagen dagegen sind keine organischen Ursachen nachweisbar. Deshalb wird die Diagnose nur dann gestellt, wenn andere Erkrankungen ausgeschlossen werden können. Die Symptome sind sehr vielfältig: Manchmal stehen Sodbrennen und Aufstoßen im Vordergrund; andere Patienten leiden stärker unter Völlegefühl, Blähungen und Übelkeit.

Je nach Schwere und Ursache der Erkrankung kommen unterschiedliche Medikamente zum Einsatz: In leichteren Fällen genügt die Neutralisierung der Magensäure mit sogenannten Antazida, beispielsweise auf Basis von Hydroxiden, Carbonaten und Silikaten des Aluminiums, Magnesiums, Calciums und Natriums (z. B. Aluminiumhydroxid, Magnesiumhydroxid, Magnesiumtrisilikat, Calciumcarbonat, Natriumbicarbonat etc.). Während aluminiumhaltige Antazida den Nachteil einer Phosphatbindung beinhalten und oftmals zu Obstipationen führen und zudem in größeren Mengen zu Aluminium-Intoxikationen (z. B. Dialyse-Enzephalopathie) führen können und zudem andere Arzneimittel, wie Tetracycline, Digoxin, Chinolone etc., unerwünschterweise binden, können calciumbasierte Antazida zu einer sogenannten Hypercalcämie, zu einer Alkalose und in schweren Fällen auch zu Niereninsuffizienz (Milch-Alkali-Syndrom) führen. Natriumbicarbonat beispielsweise kann zu einer metabolischen Alkalose führen; auch ist die Natriumbelastung nicht zu vernachlässigen, und außerdem führt die CO₂-Bildung zu einem unerwünschten Aufstoßen und kann sogar zu einer Magenruptur führen. Zwar führen die vorgenannten Antazida zu einer Neutralisierung der Magensäure, jedoch wird kein zusätzlicher Schutz für das angegriffene Gewebe bereitgestellt.

Die EP 0 506 563 A1 betrifft eine pharmazeutische Zusammensetzung in Form einer Suspension, welche neben Alginsäure und Natriumdicarbonat noch mindestens ein Viskositätsmittel aus der Gruppe von Xanthangummi, Magnesiumalginat und Glycerol enthält.

Weiterhin betrifft die wissenschaftliche Publikation Höllriegel et al., "Observation of changes in urinary excretion of thorium in humans following ingestion of a therapeutic soil", Journal of Environmental Radioactivity, 95, 2007, Seiten 149 bis 160*,* Untersuchungen zur renalen Ausscheidung des Radionuklids Thorium, wobei diesbezüglich entsprechende Harnuntersuchungen vorgenommen werden. Das Thorium wird mittels einer Heilerde verabreicht.

In schweren Fällen kommen andere, wirksamere Medikamente zum Einsatz, beispielsweise Protonenpumpenblocker (z. B. Omeprazol, Lansoprazol, Pantoprazol, Esomeprazol etc.), Histamin-H₂-Rezeptoren-Antagonisten (z. B. Ranitidin, Famotidin, Nizatidin, Cimetidin etc.), Misoprostol (ein Prostaglandin-Derivat), Sucralfat und dergleichen. Diese Medikamente können jedoch zu schwerwiegenden Nebenwirkungen führen, die von Kopfschmerzen und Diarrhoe bis hin zu Myopathien, Gastrinerhöhung, Depressionen, Lethargie und Hepatitis reichen können.

Da bei Magengeschwüren fast immer das Bakterium Helicobacter pylori zu finden ist, muß hier zusätzlich eine Eradizierung mit geeigneten Antibiotika bzw. Antibiotikakombinationen durchgeführt werden.

Der vorliegenden Erfindung liegt die Aufgabe zugrunde, eine Zusammensetzung, insbesondere pharmazeutische Zubereitung, bereitzustellen, vorzugsweise in Form eines Antazidums, welche sich insbesondere zur prophylaktischen und/oder kurativen Behandlung von Hyperazidität des Magens und damit in Zusammenhang stehenden Folgeerscheinungen und Erkrankungen (z. B. Sodbrennen, Ösophagitis, wie z. B. Refluxösophagitis, dyspeptische Beschwerden, Reizmagen, Gastritis, Ulcuserkrankungen und dergleichen) eignet und die zuvor geschilderten Nachteile des Standes der Technik zumindest weitgehend vermeidet oder aber wenigstens abschwächt.

Die Anmelderin hat nun überraschenderweise herausgefunden, daß sich die zuvor geschilderte Aufgabe dadurch lösen läßt, daß man Heilerde einerseits und Alginsäure und/oder mindestens ein physiologisch verträgliches Alginsäuresalz (Alginat) andererseits zusammen mit mindestens einer unter physiologischen Bedingungen im Magen und/oder unter Einwirkung von Säure, insbesondere Salzsäure, gasfreisetzenden Komponente kombiniert.

Gegenstand der vorliegenden Erfindung - gemäß einem **ersten** Aspekt der vorliegenden Erfindung - ist somit eine Zusammensetzung, insbesondere pharmazeutische Zubereitung, welche sich insbesondere zur prophylaktischen und/oder kurativen Behandlung von Hyperazidität und damit in Zusammenhang stehenden Folgeerscheinungen und Erkrankungen eignet und vorzugsweise in Form eines Antazidums vorliegt, wobei die Zusammensetzung - in Kombination und in jeweils wirksamen, insbesondere pharmazeutisch wirksamen Mengen und jeweils bezogen auf die Zusammensetzung -
(a) 5 bis 80 Gew.-% Heilerde; und
(b) 1 bis 70 Gew.-% Alginsäure und/oder mindestens ein physiologisch verträgliches Alginsäuresalz;
   zusammen mit
(c) 0,1 bis 50 Gew.-% mindestens einer unter physiologischen Bedingungen im Magen und/oder unter Einwirkung von Säure, insbesondere Salzsäure, gasfreisetzenden Komponente, wobei die gasfreisetzende Komponente ein Carbonat und/oder Hydrogencarbonat ist,
enthält.

Der Begriff "pharmazeutische Zusammensetzung" bzw. "pharmazeutische Zubereitung", wie er im Rahmen der vorliegenden Erfindung verwendet wird, ist sehr breit zu verstehen und umfaßt nicht nur pharmazeutische Präparate bzw. Pharmazeutika und Arzneimittel als solche, sondern auch sogenannte Medizinprodukte.

Weiterhin bezieht sich der Begriff "physiologische Bedingungen im Magen", wie er im Rahmen der vorliegenden Erfindung verwendet wird, insbesondere auf die physiologischen Bedingungen bzw. das Milieu im Magenlumen bzw. im Magensaft. In diesem Zusammenhang fokussiert der Begriff "physiologische Bedingungen im Magen" vorrangig auf die Azidität des Magensaftes, welcher aufgrund der Sekretion von in den Belegzellen der Magenschleimhaut produzierter Salzsäure einen pH-Wert von etwa 1 bis 1,5 im nüchternen Zustand und etwa 2 bis 4 bei mit Nahrung gefülltem Magen aufweist.

Die Anmelderin hat überraschenderweise herausgefunden, daß durch die gezielte Kombination von Heilerde einerseits und Alginsäure bzw. mindestens einem physiologisch verträglichen Alginsäuresalz andererseits zusammen mit mindestens einer unter physiologischen Bedingungen im Magen bzw. unter Einwirkung von Säure, insbesondere Salzsäure, gasfreisetzenden Komponente (z. B. in erfindungsgemäß besonders bevorzugter Weise Natriumhydrogencarbonat) ein wirksames Therapeutikum für die Behandlung von Hyperazidität des Magens und damit in Zusammenhang stehender Folgeerscheinungen und -erkrankungen bereitgestellt werden kann, welches gut verträglich und nebenwirkungsfrei bzw. nahezu nebenwirkungsfrei ist.

Bei der im Rahmen der vorliegenden Erfindung eingesetzten Heilerde handelt es sich im allgemeinen um ein insbesondere medizinisches Pulver, welches beispielsweise aus eiszeitlichen Lößablagerungen gewonnen und durch spezielle Verfahrensweisen bearbeitet werden kann. Im medizinischen Sinne handelt es sich bei der erfindungsgemäß eingesetzten Heilerde insbesondere um eine Tonerde, die auch - um sie von anderen nichtmedizinischen Erden zu unterscheiden - als Tonheilerde bezeichnet werden kann. Aufgrund ihrer spezifischen Zusammensetzung ist Heilerde - wie nachfolgend noch angeführt - imstande, im Rahmen der erfindungsgemäßen Verwendung überschüssige Magensäure zu binden. Aufgrund ihrer großen inneren Oberfläche kann die Heilerde - ohne sich auf diese Theorie beschränken zu wollen - im Rahmen des erfindungsgemäßen Konzeptes mit der Magensäure, insbesondere Salzsäure, in Wechselwirkung treten und diese gewissermaßen binden bzw. neutralisieren. Aufgrund ihrer spezifischen Eigenschaften führt der zweckgerichtete Einsatz von Heilerde im Rahmen der erfindungsgemäßen Zusammensetzung bei guter Verträglichkeit zu einer besonders hohen antaziden Wirksamkeit der resultierenden erfindungsgemäßen Zusammensetzung.

Ohne sich auf diese Theorie beschränken zu wollen, kann die Neutralisierung bzw. Bindung der übermäßigen Salzsäure im Magensaft bzw. im Magenlumen durch die Heilerde vorrangig aufgrund physikalischer bzw. physikochemischer Prozesse, wie Adsorptions- und/oder Absorptionsprozesse, erklärt werden. In diesem Zusammenhang hat sich die Verwendung von Heilerde als besonders vorteilhaft erwiesen, da diese aufgrund ihrer spezifischen Struktur mit einer hohen inneren Oberfläche zu einer hervorragenden Neutralisierung bzw. Bindung und/oder Aufnahme von Salzsäure führt - und dies bei gleichzeitig guter Verträglichkeit.

Was die erfindungsgemäß eingesetzte Heilerde weiterhin anbelangt, so kann diese mindestens einen Bestandteil aus der Gruppe von Silikaten, Dreischichttonmineralen, insbesondere Illit, Smectid und/oder Colorit, Feldspaten, Kalzit und Dolomit enthalten.

Beispielsweise kann die erfindungsgemäß eingesetzte Heilerde Silikate des Aluminiums, Magnesiums und Calciums sowie deren Mischungen und Mischverbindungen, insbesondere Erdalkalihydroxide, -carbonate und/oder -silikate, bevorzugt des Magnesiums und des Calciums, ganz besonders bevorzugt des Magnesiums, enthalten.

Weiterhin kann die erfindungsgemäß eingesetzte Heilerde, insbesondere in gebundener Form, mindestens ein chemisches Element, ausgewählt aus der Gruppe von Sauerstoff, Silizium, Calcium, Aluminium, Eisen, Kalium, Magnesium, Natrium, Titan und Phosphor, enthalten.

Weiterhin kann die erfindungsgemäß eingesetzte Heilerde auch Spurenelemente, insbesondere ausgewählt aus der Gruppe von Kupfer, Mangan, Nickel, Selen und Zink, enthalten.

Im allgemeinen sind die Elemente in den Mineralien der Heilerde insbesondere chemisch gebunden, wobei jedoch einzelne Elemente bei Applikation der erfindungsgemäßen Zusammensetzung im Magenmilieu insbesondere durch Einfluß der Magensäure mitunter in geringen Mengen aus der Heilerde herausgelöst werden können, was beispielsweise auf Calcium, Kalium und Magnesium zutreffen kann, während z. B. Aluminium beispielsweise in sogenannten Alumosilikaten schwerlöslich gebunden ist. Auf diese Weise kann eine zusätzliche Versorgung des Körpers mit Mineralstoffen sichergestellt werden.

Im allgemeinen handelt es sich bei der erfindungsgemäß eingesetzten Heilerde um ein reines Naturprodukt, so daß in bezug auf die erfindungsgemäße Zusammensetzung eine besonders gute Verträglichkeit resultiert.

Was die im Rahmen der erfindungsgemäßen Zusammensetzung eingesetzte Heilerde anbelangt, so sind Heilerden als solche dem Fachmann wohlbekannt, so daß der Fachmann jederzeit in der Lage ist, die im Rahmen der vorliegenden Erfindung einsetzbare Heilerde entsprechend auszuwählen bzw. aufzuarbeiten.

Wegen der Nebenwirkungen von Aluminium bzw. Aluminiumverbindungen (z. B. Phosphatbindungen, Obstipationen und Aluminium-Intoxikationen) ist der Einsatz von Heilerden im Rahmen der vorliegenden Erfindung besonders vorteilhaft, da eine Freisetzung von Aluminium bzw. Aluminiumverbindungen zumindest im wesentlichen vermieden wird.

Die erfindungsgemäß eingesetzte Heilerde zeichnet sich zudem dadurch aus, daß sie über einen sehr langen Zeitraum im Magen wirkt, da die im Magensaft befindliche Salzsäure über einen verlängerten Zeitraum von der Heilerde aufgrund ihrer spezifischen Eigenschaften insbesondere im Hinblick auf ihre große innere Oberfläche gebunden bzw. neutralisiert wird. Die Heilerde wirkt demnach über einen längeren Zeitraum antazid und ist dabei in der Lage, überschüssige Magensäure langsam und damit physiologisch zu binden, um auf diese Weise einen möglichen Säure-Rebound zu verhindern.

Diesbezüglich unterscheidet sich Heilerde deutlich von anderen antazid wirksamen Salzen bzw. Substanzen. In den erfindungsgemäß vorgesehenen Mengen führt die Heilerde dazu, daß - insbesondere im Unterschied zu Aluminiumhydroxidverbindungen - Obstipationen in wirksamer Weise verhindern werden. Im Gegensatz hierzu regt Heilerde sogar noch die Darmperistaltik in positiver Weise an.

Das der vorliegenden Erfindung zugrundeliegende Prinzip besteht insgesamt darin, die Heilerde einerseits mit Alginsäure bzw. mindestens einem physiologisch verträglichen Alginsäuresalz zu kombinieren, wobei - wie nachfolgend angeführt - durch die weitere Zugabe einer unter physiologischen Bedingungen im Magen und/oder Einwirkung von Säure, insbesondere Salzsäure, gasfreisetzenden Komponente, insbesondere Natriumhydrogencarbonat, die Wirkung der erfindungsgemäßen Zusammensetzung weiter verbessert werden kann.

Die Alginsäure bzw. Alginate wird von Braunalgen in den Zellen gebildet und stellt in der Alge das strukturgebende Element dar. Die interzelluläre Gelmatrix verleitet der Alge sowohl Flexibilität als auch Festigkeit. Alginsäure ist unter anderem ein Nebenprodukt bei der Gewinnung von Iod aus Meeresalgen im Naßverfahren. Für die Verwendung in der Lebensmittel-, Pharma- und Kosmetikindustrie wird es gezielt aus Braunalgen extrahiert. Die Salze der Alginsäure werden im allgemeinen als Alginate bezeichnet. Alginate finden vor allem als Verdickungs- oder Geliermittel Verwendung. Alginsäure bzw. die Alginate sind Polysaccharide, welche aus 1,4-verknüpfter α-L-Guluronsäure (G) und β-D-Mannuronsäure (M) bestehen, wobei homopolymere Bereiche gebildet werden, in denen Mannuronsäure oder Guluronsäure als Block vorliegen, wobei diese Blöcke auch als GG- oder MM-Blöcke bezeichnet werden; im Bereich der GG- und MM-Blöcke kommt es zu einer Faltstruktur, die bei der Gelierung eine wesentliche Rolle spielt, insbesondere die GG-Blöcke bilden eine regelmäßige Zickzack-Struktur aus.

Im Rahmen der erfindungsgemäßen pharmazeutischen Zubereitung wirkt die Alginsäure bzw. das Alginat wie folgt: Bei Kontakt mit der Magensäure nimmt die Alginsäure bzw. das Alginat Wasser auf und bildet eine viskose Schicht. Im Magen beginnt die Alginsäure, eine viskose und schaumige Suspension zu bilden. Diese Schaumschranke aus Alginsäure bzw. Alginaten befindet sich auf dem spezifisch schwereren Mageninhalt und reduziert auf diese Weise rein physikalisch die Anzahl der gastroösophagialen Refluxepisoden. Wird der Mageninhalt kardiawärts gepreßt, schützt der Schaum aus Alginsäure bzw. Alginaten die Speiseröhre vor dem Kontakt mit der Magensäure. Der pH-Wert unterhalb der physikalischen Schaumschranke bleibt durch die Alginsäure selbst mit ca. 1 unangetastet, während die Schaumschranke selbst auf eine nahezu neutralen pH-Wert eingestellt wird - infolge der Anwesenheit des Magensäure neutralisierenden bzw. bindenden Wirkstoffs. Die auf dem Mageninhalt schwimmende Schaumschranke sinkt mit fortschreitender Entleerung des Magens ab und kommt am Pylorus in Kontakt mit alkalischen Valenzen des Duodenums. Bei ansteigendem pH-Wert löst sich die Schaumschranke auf und wird praktisch unverdaut ausgeschieden. Die normalen Verdauungsvorgänge bleiben unbeeinflußt, d. h. die Alginsäure bzw. die Alginate wirken rein physikalisch und werden nicht resorbiert. Die gleichzeitig vorhandene, Magensäure bindende bzw. neutralisierende Heilerde ist dagegen in der Lage, die überschüssige Magensäure zu binden bzw. zu neutralisieren. Zudem bewirkt die Heilerde, daß die alginsäurehaltige physikalische Schaumschranke im wesentlichen pH-neutral ist.

Für die Zwecke der vorliegenden Erfindung besonders bevorzugt ist eine Alginsäure bzw. ein Alginat mit einer relativen Molmasse von 20.000 bis 250.000 Dalton, insbesondere 30.000 bis 220.000 Dalton, besonders bevorzugt 40.000 bis 200.000 Dalton.

Die zuvor beschriebene Wirkung der Alginsäure kann durch die gezielte Verwendung eines unter physiologischen Bedingungen im Magen bzw. unter Einwirkung von Säure, insbesondere Salzsäure, gasfreisetzenden Komponente noch deutlich verbessert bzw. gesteigert werden. Denn die gasfreisetzende Komponente führt bei Einnahme der Zusammensetzung und Freisetzung der Komponenten im Magenlumen bzw. im Magensaft zu einer Gasbildung, beispielsweise im Rahmen einer chemischen Reaktion, so daß aufgrund der gezielten Gasbildung die Schaumbildung in bezug auf die Alginsäure bzw. dem physiologisch verträglichen Alginsäuresalz signifikant verbessert wird. Es resultiert somit durch den Einsatz der gasfreisetzenden Komponente eine signifikante Verbesserung der durch die Alginsäure ausgebildeten Schaumschranke, so daß die zuvor im Zusammenhang mit der Schaumschranke beschriebenen Effekte bzw. Wirkungen deutlich verbessert werden können. Wie nachfolgend noch angeführt, hat sich im Rahmen der vorliegenden Erfindung insbesondere eine Kombination von Heilerde einerseits und Alginsäure bzw. einem physiologisch verträglichen Alginsäuresalz andererseits zusammen mit einem Erdalkalicarbonat und/oder Erdalkalihydrogencarbonat bzw. einem Alkalicarbonat und/oder Alkalihydrogencarbonat bewährt. Im Rahmen der vorliegenden Erfindung ist jedoch der Einsatz einer Vielzahl von unter den zuvor genannten Bedingungen gasfreisetzenden Komponenten möglich. Dabei ist insbesondere zu beachten, daß das freigesetzte Gas physiologisch gut verträglich und nicht zu einer gesundheitlichen Beeinträchtigung führt bzw. in den freigesetzten Mengen als solches nicht schädlich ist. In diesem Zusammenhang handelt es sich erfindungsgemäß bei der gasfreisetzenden Komponente um eine CO₂-freisetzende Komponente. Auch die weiteren bei der Gasbildungsreaktion gebildeten Stoffe bzw. Komponenten sollten im allgemeinen gut verträglich sein.

Durch die gezielte Kombination von Heilerde einerseits und Alginsäure bzw. einem physiologisch verträglichen Alginsäuresalz zusammen mit der im Magen gasfreisetzenden Komponente ist erstmals ein nahezu nebenwirkungsfreies Therapeutikum für die alleinige oder begleitende Behandlung von Hyperazidität des Magens und damit in Zusammenhang stehenden Folgeerscheinungen und -erkrankungen bereitgestellt worden, welches gleichzeitig die eingangs geschilderten Nachteile des Standes der Technik zumindest weitgehend vermeidet oder aber wenigstens abschwächt.

Wie zuvor beschrieben, enthält die erfindungsgemäße pharmazeutische Zusammensetzung eine Kombination der Wirkstoffe (a) Heilerde einerseits und (b) Alginsäure bzw. Alginsäuresalz(e) andererseits zusammen mit (c) mindestens einer unter physiologischen Bedingungen im Magen bzw. unter Einwirkung von Säure, insbesondere Salzsäure, gasfreisetzenden Komponente, wobei es sich bei der gasfreisetzenden Komponente erfindungsgemäß um ein Carbonat und/oder Hydrogencarbonat handelt.

Darüber hinaus enthält die erfindungsgemäße Zusammensetzung üblicherweise zusätzlich mindestens einen pharmazeutischen Träger bzw. Exzipienten.

Im allgemeinen liegt die erfindungsgemäße Zusammensetzung in peroral verabreichbarer Applikationsform vor. Dabei kann die erfindungsgemäße Zusammensetzung entweder in fester oder aber in flüssiger Dosierungsform vorliegen.

Im Fall einer festen Dosierungsform liegt die erfindungsgemäße Zusammensetzung insbesondere als Pulver, Granulat oder Tablette, vorzugsweise als Tablette, besonders bevorzugt als Kautablette, vor. Im Fall einer flüssigen Dosierungsform liegt die erfindungsgemäße Zusammensetzung insbesondere als Suspension (z. B. verpackt in einem dosierfertigen Suspensionsbeutel für die einmalige Anwendung) vor. Die erfindungsgemäße Zusammensetzung kann beispielsweise in Form eines Pulvers oder Granulats auch in Dosierbeuteln eingebracht vorliegen, wobei in diesem Fall das Pulver oder das Granulat nach Entnahme aus dem Dosierbeutel beispielsweise auch zur Herstellung eines Instantgetränkes mit Wasser angerührt werden kann.

Die Menge an den jeweiligen Inhaltsstoffen in der erfindungsgemäßen Zusammensetzung kann in weiten Bereichen variieren. Es versteht sich von selbst, daß bei den nachfolgenden Mengenangaben im Fall von Prozentangaben die Inhaltsstoffe so zu kombinieren sind, daß die Prozentangaben unter Einbezug weiterer Inhaltsstoffe insgesamt stets 100 % ergeben. Im übrigen gilt, daß der Fachmann anwendungsbezogen oder einzelfallbedingt von den nachfolgend aufgeführten Mengenangaben abweichen kann, ohne daß er den Rahmen der vorliegenden Erfindung verläßt.

Was die Menge an (a) Heilerde in der erfindungsgemäßen Zusammensetzung anbelangt, so kann diese in weiten Bereichen variieren: Die erfindungsgemäße Zusammensetzung enthält (a) die Heilerde in Mengen von 5 bis 80 Gew.-%, insbesondere 10 bis 70 Gew.-%, vorzugsweise 15 bis 50 Gew.-%, besonders bevorzugt 15 bis 40 Gew.-%, ganz besonders bevorzugt 20 bis 30 Gew.-%, bezogen auf die Zusammensetzung.

Was die Alginsäure und/oder das Alginsäuresalz anbelangt, so kann deren bzw. dessen Mengen gleichermaßen in weiteren Grenzen variieren. Die erfindungsgemäße Zusammensetzung enthält die Alginsäure und/oder das Alginsäuresalz in Mengen von insgesamt 1 bis 70 Gew.-%, insbesondere 5 bis 60 Gew.-%, vorzugsweise 10 bis 55 Gew.-%, besonders bevorzugt 15 bis 50 Gew.-%, ganz besonders bevorzugt 20 bis 40 Gew.-%, bezogen auf die Zusammensetzung

Neben den relativen bzw. prozentualen Mengenanteilen der Wirk- bzw. Inhaltsstoffe in der erfindungsgemäßen Zusammensetzung bestimmt auch deren massebezogenes Verhältnis zueinander die Wirksamkeit der erfindungsgemäßen pharmazeutischen Zusammensetzung. Im allgemeinen enthält die erfindungsgemäße pharmazeutische Zusammensetzung die Wirkstoffe (a) "Heilerde" und (b) "Alginsäure und/oder mindestens ein physiologisch verträgliches Alginsäuresalz (Alginat)" in einem massenbezogenen Verhältnis von (a) / (b) im Bereich von 0,1 - 10/0,1 - 10, insbesondere 0,5 - 5 / 0,5 - 5, vorzugsweise 0,75 - 3 / 0,75 - 3, bevorzugt 1 - 2 / 1 - 2.

Neben dem relativen Mengenanteil und dem massenbezogenen Mengenverhältnis spielt auch die absolute Konzentration bzw. die absolute Menge pro Dosiereinheit eine entscheidende Rolle in bezug auf die Wirksamkeit der erfindungsgemäßen pharmazeutischen Zusammensetzung:

Im allgemeinen enthält die erfindungsgemäße pharmazeutische Zusammensetzung die (a) Heilerde pro Dosiereinheit, insbesondere je Tablette oder je Dosierbeutel oder je Suspensionsbeutel, in Mengen von 10 bis 5.000 mg, insbesondere 50 bis 3.000 mg, vorzugsweise 100 bis 2.000 mg, besonders bevorzugt 200 bis 1.500 mg, ganz besonders bevorzugt 400 bis 1.000 mg.

Was die Alginsäure und/oder das Alginsäuresalz anbelangt, so kann die erfindungsgemäße pharmazeutische Zusammensetzung die Alginsäure und/oder das Alginsäuresalz pro Dosiereinheit, insbesondere je Tablette oder je Dosierbeutel oder je Suspensionsbeutel, in Mengen von insgesamt 10 bis 1.500 mg, insbesondere 50 bis 1.250 mg, vorzugsweise 75 bis 1.000 mg, besonders bevorzugt 100 bis 800 mg, ganz besonders bevorzugt 150 bis 600 mg, enthalten.

Was die (c) unter physiologischen Bedingungen im Magen und/oder unter Einwirkung von Säure, insbesondere Salzsäure, gasfreisetzende Komponente anbelangt, so ist diese erfindungsgemäß ein Carbonat und/oder Hydrogencarbonat.

In diesem Zusammenhang kann die (c) unter physiologischen Bedingungen im Magen und/oder unter Einwirkung von Säure, insbesondere Salzsäure, gasfreisetzende Komponente insbesondere ein Erdalkalicarbonat und/oder Erdalkalihydrogencarbonat, insbesondere des Magnesiums und/oder des Calciums, sein.

Gleichermaßen ist es im Rahmen der vorliegenden Erfindung möglich, daß die (c) unter physiologischen Bedingungen im Magen und/oder unter Einwirkung von Säure, insbesondere Salzsäure, gasfreisetzende Komponente ein Alkalicarbonat und/oder Alkalihydrogencarbonat, insbesondere des Kaliums und/oder des Natriums, vorzugsweise des Natriums, ist. In diesem Zusammenhang hat die Anmelderin in völlig überraschender Weise gefunden, daß erfindungsgemäß besonders gute Ergebnisse hinsichtlich der Schaumbildung gefunden werden, wenn (c) die unter physiologischen Bedingungen im Magen und/oder unter Einwirkung von Säure, insbesondere Salzsäure, gasfreisetzende Komponente Natriumhydrogencarbonat ist.

In diesem Zusammenhang resultiert bei der Verwendung von Natriumhydrogencarbonat - ohne sich auf diese Theorie beschränken zu wollen - im Magen unter Säureeinfluß, insbesondere Salzsäureeinfluß, die Bildung von Kohlendioxid (CO₂) als zur Aufschäumung der Alginsäure bzw. des Alginats dienendes Gas, wobei im Rahmen der zugrundeliegenden chemischen Reaktionen zudem lediglich die physiologisch unbedenklichen Verbindungen Natriumchlorid und Wasser entstehen. Da die gasfreisetzende Reaktion von Natriumhydrogencarbonat unter Verbrauch von Säure, insbesondere Salzsäure, stattfindet, besteht ein weiterer Vorteil darin, daß durch die Gasbildungsreaktion zusätzlich überflüssige Salzsäure im Magen abgebaut wird. Das bei der Verwendung von Natriumhydrogencarbonat im Rahmen der Gasbildungsreaktion entstehende Kohlendioxid ist zudem in den freigesetzten Mengen gesundheitlich unbedenklich und führt zu einer guten Aufschäumung und somit zu einer verbesserten Leistungsfähigkeit der Alginsäure- bzw. Alginatschaumschranke.

Was die Menge an (c) der unter physiologischen Bedingungen im Magen und/oder unter Einwirkung von Säure, insbesondere Salzsäure, gasfreisetzende Komponente anbelangt, so kann diese in weiten Bereichen variieren. Die erfindungsgemäße Zusammensetzung enthält die (c) unter physiologischen Bedingungen im Magen und/oder unter Einwirkung von Säure, insbesondere Salzsäure, gasfreisetzende Komponente in Mengen von insgesamt 0,1 bis 50 Gew.-%, insbesondere 0,5 bis 30 Gew.-%, vorzugsweise 1 bis 20 Gew.-%, besonders bevorzugt 1,5 bis 10 Gew.-%, ganz besonders bevorzugt 2 bis 10 Gew.-%, bezogen auf die Zusammensetzung.

Wie zuvor für die Komponenten (a) und (b) geschildert, spielt auch hinsichtlich der Komponente (c) die absolute Menge pro Dosiereinheit eine entscheidende Rolle in bezug auf die Wirksamkeit der erfindungsgemäßen pharmazeutischen Zusammensetzung: Im allgemeinen enthält die erfindungsgemäße Zusammensetzung die (c) unter physiologischen Bedingungen im Magen und/oder unter Einwirkung von Säure, insbesondere Salzsäure, gasfreisetzende Komponente pro Dosiereinheit, insbesondere je Tablette oder je Dosierbeutel oder je Suspensionsbeutel, in Mengen von 1 bis 500 mg, insbesondere 5 bis 300 mg, vorzugsweise 10 bis 200 mg, besonders bevorzugt 20 bis 150 mg, ganz besonders bevorzugt 30 bis 130 mg.

Auch das massenbezogene Verhältnis der Wirkstoffkomponenten (a), (b) und (c) zueinander spielt hinsichtlich der Wirksamkeit der erfindungsgemäßen pharmazeutischen Zusammensetzung eine große Rolle. Im allgemeinen enthält die erfindungsgemäße pharmazeutische Zusammensetzung die Wirkstoffkomponenten (a) "Heilerde", (b) "Alginsäure und/oder mindestens ein physiologisch verträgliches Alginsäuresalz (Alginat)" und (c) "unter physiologischen Bedingungen im Magen und/oder unter Einwirkung von Säure, insbesondere Salzsäure, gasfreisetzende Komponente" in einem massenbezogenen Verhältnis von (a) / (b) / (c) im Bereich von 0,01 - 50 / 0,01 - 20 / 0,1 - 10, insbesondere 0,1 - 20 / 0,1 - 10 / 0,1 - 5, vorzugsweise 0,1 - 15 / 0,5 - 8 / 0,1 - 3, bevorzugt 5 - 10 / 5 / 1.

Gemäß einer besonderen Ausführungsform betrifft die vorliegende Erfindung eine Zusammensetzung, insbesondere pharmazeutische Zubereitung, insbesondere zur prophylaktischen und/oder kurativen Behandlung von Hyperazidität und damit in Zusammenhang stehenden Folgeerscheinungen und Erkrankungen, vorzugsweise in Form eines Antazidums, wie zuvor beschrieben, wobei die Zusammensetzung - in Kombination und in jeweils wirksamen, insbesondere pharmazeutisch wirksamen Mengen und jeweils bezogen auf die Zusammensetzung -
(a) Heilerde, insbesondere in Mengen von 5 bis 80 Gew.-%, insbesondere 10 bis 70 Gew.-%, vorzugsweise 15 bis 50 Gew.-%, besonders bevorzugt 15 bis 40 Gew.-%, ganz besonders bevorzugt 20 bis 30 Gew.-%, insbesondere wobei (a) die Heilerde mindestens einen Bestandteil aus der Gruppe von Silikaten, Dreischichttonmineralen, insbesondere Illit, Smectid und/oder Colorit, Feldspaten, Kalzit und Dolomit enthält;
(b) Alginsäure und/oder mindestens ein physiologisch verträgliches Alginsäuresalz, insbesondere in Mengen von insgesamt 1 bis 70 Gew.-%, insbesondere 5 bis 60 Gew.-%, vorzugsweise 10 bis 55 Gew.-%, besonders bevorzugt 15 bis 50 Gew.-%, ganz besonders bevorzugt 20 bis 40 Gew.-%;
   zusammen mit
(c) mindestens einer unter physiologischen Bedingungen im Magen und/oder unter Einwirkung von Säure, insbesondere Salzsäure, gasfreisetzenden Komponente, insbesondere wobei die Komponente (c) ein Erdalkalicarbonat und/oder Erdalkalihydrogencarbonat, insbesondere des Magnesiums und/oder des Calciums, ist und/oder insbesondere wobei die Komponente (c) ein Alkalicarbonat und/oder Alkalihydrogencarbonat, insbesondere des Kaliums und/oder des Natrium, vorzugsweise des Natriums, ist, und/oder insbesondere wobei die Komponente (c) Natriumhydrogencarbonat ist, und/oder insbesondere wobei die Zusammensetzung die Komponente (c) in Mengen von insgesamt 0,1 bis 50 Gew.-%, insbesondere 0,5 bis 30 Gew.-%, vorzugsweise 1 bis 20 Gew.-%, besonders bevorzugt 1,5 bis 10 Gew.-%, ganz besonders bevorzugt 2 bis 10 Gew.-%, bezogen auf die Zusammensetzung, enthält;
   enthält.
   Gemäß einer anderen besonderen Ausführungsform der vorliegenden Erfindung betrifft die vorliegende Erfindung eine Zusammensetzung, insbesondere pharmazeutische Zubereitung, insbesondere zur prophylaktischen und/oder kurativen Behandlung von Hyperazidität und damit in Zusammenhang stehenden Folgeerscheinungen und Erkrankungen, vorzugsweise in Form eines Antazidums, wie zuvor beschrieben, wobei die Zusammensetzung - in Kombination und in jeweils wirksamen, insbesondere pharmazeutisch wirksamen Mengen -
   (a) Heilerde, insbesondere wobei (a) die Heilerde mindestens einen Bestandteil aus der Gruppe von Silikaten, Dreischichttonmineralen, insbesondere Illit, Smectid und/oder Colorit, Feldspaten, Kalzit und Dolomit enthält, insbesondere wobei die Zusammensetzung die (a) Heilerde pro Dosiereinheit, insbesondere je Tablette oder je Dosierbeutel oder je Suspensionsbeutel, in Mengen von 10 bis 5.000 mg, insbesondere 50 bis 3.000 mg, vorzugsweise 100 bis 2.000 mg, besonders bevorzugt 200 bis 1.500 mg, ganz besonders bevorzugt 400 bis 1.000 mg, enthält;
   (b) Alginsäure und/oder mindestens ein physiologisch verträgliches Alginsäuresalz, insbesondere wobei die Zusammensetzung (b) die Alginsäure und/oder das Alginsäuresalz pro Dosiereinheit, insbesondere je Tablette oder je Dosierbeutel oder je Suspensionsbeutel, in Mengen von insgesamt 10 bis 1.500 mg, insbesondere 50 bis 1.250 mg, vorzugsweise 75 bis 1.000 mg, besonders bevorzugt 100 bis 800 mg, ganz besonders bevorzugt 150 bis 600 mg, enthält;
      zusammen mit
   (c) mindestens einer unter physiologischen Bedingungen im Magen und/oder unter Einwirkung von Säure, insbesondere Salzsäure, gasfreisetzenden Komponente, insbesondere wobei die Komponente (c) ein Erdalkalicarbonat und/oder Erdalkalihydrogencarbonat, insbesondere des Magnesiums und/oder des Calciums, ist und/oder insbesondere wobei die Komponente (c) ein Alkalicarbonat und/oder Alkalihydrogencarbonat, insbesondere des Kaliums und/oder des Natrium, vorzugsweise des Natriums, ist, und/oder insbesondere wobei die Komponente (c) Natriumhydrogencarbonat ist, und/oder insbesondere wobei die Zusammensetzung die Komponente (c), insbesondere je Tablette oder je Dosierbeutel oder je Suspensionsbeutel, in Mengen von 1 bis 500 mg, insbesondere 5 bis 300 mg, vorzugsweise 10 bis 200 mg, besonders bevorzugt 20 bis 150 mg, ganz besonders bevorzugt 30 bis 130 mg, enthält;
   enthält.

Gemäß einer wiederum anderen besonderen Ausführungsform der vorliegenden Erfindung betrifft die vorliegende Erfindung eine Zusammensetzung, insbesondere pharmazeutische Zubereitung, insbesondere zur prophylaktischen und/oder kurativen Behandlung von Hyperazidität und damit in Zusammenhang stehenden Folgeerscheinungen und Erkrankungen, vorzugsweise in Form eines Antazidums, wie zuvor beschrieben, wobei die Zusammensetzung - in Kombination und in jeweils wirksamen, insbesondere pharmazeutisch wirksamen Mengen pro Dosiereinheit, insbesondere je Tablette oder je Dosierbeutel oder je Suspensionsbeutel -
(a) Heilerde, insbesondere in Mengen von 400 bis 1.000 mg;
(b) Alginsäure und/oder mindestens ein physiologisch verträgliches Alginsäuresalz, insbesondere in Mengen von insgesamt 150 bis 600 mg;
   zusammen mit
(c) Natriumhydrogencarbonat, insbesondere in Mengen von 30 bis 130 mg;
enthält.

In bezug auf die zuvor geschilderten besonderen Ausführungsformen der vorliegenden Erfindung gelten die vorstehenden Ausführungen zu den allgemeinen Ausführungsformen entsprechend.

Neben den vorgenannten Wirk- und Inhaltsstoffen kann die erfindungsgemäße Zusammensetzung außerdem weitere Inhaltsstoffe enthalten. Diese können insbesondere ausgewählt sein aus Zusatz- und/oder Hilfsstoffen, wie beispielsweise Füll-, Streck-, Binde-, Netz-, Stabilisierungs-, Färbe-, Puffer-, Riech-, Geschmacks-, Süßungs-, Aroma-, Verarbeitungshilfs- und/oder Konservierungsstoffen und -mitteln.

Im allgemeinen liegt die erfindungsgemäße pharmazeutische Zusammensetzung, wie sie vorstehend beschrieben ist, in Dosiereinheiten, insbesondere Tabletten, Dosierbeuteln von Pulvern, Granulaten oder dergleichen (z. B. Pulver- oder Granulatbeutel), von je 0,1 bis 10 g, insbesondere 0,5 bis 8 g, vorzugsweise 0,5 bis 6 g, bevorzugt 1 bis 5 g, vor.

Gleichermaßen ist es aber auch möglich, die erfindungsgemäße Zusammensetzung in Form einer Suspension zu formulieren, beispielsweise unter Verwendung hierfür geeigneter Mengen an Wasser. Diesbezüglich kann die erfindungsgemäße Zusammensetzung in Dosiereinheiten, insbesondere Suspensionsbeuteln, von je 2 bis 100 g, insbesondere 5 bis 60 g, vorzugsweise 8 bis 50 g, bevorzugt 10 bis 40 g, vorliegen.

Die Dosiereinheiten der erfindungsgemäßen Zusammensetzung sind vorteilhafterweise nach dem Essen, insbesondere ein bis zwei Stunden nach dem Essen, und/oder vor dem Nachtschlaf einzunehmen, und zwar jeweils in Mengen von ein bis zwei Dosiereinheiten, und zwar in Abhängigkeit von der Schwere des Krankheitsbildes.

Die zuvor beschriebene pharmazeutische Zusammensetzung ist ein sicheres Therapeutikum zur prophylaktischen und/oder kurativen, insbesondere symptomatischen Behandlung von Hyperazidität des Magens und damit in Zusammenhang stehenden Folgeerscheinungen und Folgeerkrankungen der eingangs genannten Art. Die erfindungsgemäße Zusammensetzung führt zu keinen bzw. nahezu keinen systemischen Nebenwirkungen, gewährleistet aber dennoch eine sichere symptomatische Therapie der säurebedingten Magen- und Speiseröhrenerkrankungen - entweder als Monotherapeutikum oder begleitend zu anderen Therapeutika der eingangs genannten Art.

Zudem betrifft die vorliegende Erfindung - gemäß einem **zweiten** Aspekt der vorliegenden Erfindung - die Verwendung der erfindungsgemäßen pharmazeutischen Zusammensetzung. In bezug auf die erfindungsgemäße Verwendung kann - zur Vermeidung unnötiger Wiederholungen - auf die vorstehenden Ausführungen zu der erfindungsgemäßen Zusammensetzung selbst verwiesen werden, welche in bezug auf die erfindungsgemäße Verwendung entsprechend gelten.

Insbesondere betrifft die vorliegende Erfindung die Verwendung der erfindungsgemäßen Zusammensetzung, wie zuvor beschrieben, als Antazidum.

Des weiteren betrifft die vorliegende Erfindung die Verwendung der erfindungsgemäßen Zusammensetzung, wie zuvor beschrieben, zur prophylaktischen und/oder kurativen, insbesondere symptomatischen prophylaktischen und/oder kurativen Behandlung bzw. zur Herstellung eines Arzneimittels zur prophylaktischen und/oder kurativen, insbesondere symptomatischen prophylaktischen und/oder kurativen Behandlung von Hyperazidität (d. h. Hyperazidität des Magens) und damit in Zusammenhang stehenden Folgeerscheinungen und -erkrankungen.

Insbesondere betrifft die vorliegende Erfindung die Verwendung der zuvor beschriebenen erfindungsgemäßen Zusammensetzung zur prophylaktischen und/oder kurativen, insbesondere symptomatischen prophylaktischen und/oder kurativen Behandlung bzw. zur Herstellung eines Arzneimittels zur prophylaktischen und/oder kurativen, insbesondere symptomatischen prophylaktischen und/oder kurativen Behandlung von Sodbrennen, Ösophagitis (z. B. Refluxösophagitis), dyspeptischen Beschwerden, Reizmagen, Gastritis, Ulcuserkrankungen und dergleichen.

Bei der erfindungsgemäßen Verwendung wird die erfindungsgemäße Zusammensetzung üblicherweise nach einer Mahlzeit und/oder vor dem Nachtschlaf appliziert. Diesbezüglich kann auf die vorstehenden Ausführungen verwiesen werden.

Mit der erfindungsgemäßen Zusammensetzung wird erstmals ein nebenwirkungsfreies bzw. nahezu nebenwirkungsfreies, gut verträgliches Therapeutikum zur prophylaktischen und/oder kurativen Behandlung von Hyperazidität des Magens und damit in Zusammenhang stehenden Folgeerscheinungen und -erkrankungen der eingangs genannten Art bereitgestellt, welches auf einer Kombination von Heilerde einerseits und Alginsäure bzw. einem physiologisch verträglichen Alginsäuresalz andererseits zusammen mit mindestens einer unter physiologischen Bedingungen im Magen bzw. unter Einwirkung von Säure, wie Salzsäure, gasfreisetzende Komponente beruht und eine hohe Langzeitwirkung aufweist.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die nachfolgenden Ausführungsbeispiele dienen lediglich der Veranschaulichung der vorliegenden Erfindung,

### Ausführungsbeispiele:

### 1. Zusammensetzung A (erfindungsgemäß):

In an sich bekannter Weise wird eine erfindungsgemäße Zusammensetzung in Form von Magentabletten hergestellt:

| **Inhaltsstoffe** | **Menge in g pro Charge (Wirkstoffgranulat bzw. -pulver)** | **Menge pro Tablette in mg** |
|---|---|---|
| Heilerde | 64,94 | 200,00 |
| Alginsäure | 113,64 | 350,00 |
| Natriumhydrogencarbonat | 38,96 | 120,00 |
| Cellulose | 73,38 | 226,00 |
| Siliciumdioxid (Kieselsäure) | 5,19 | 16,00 |
| Gleitmittel | 3,90 | 12,00 |
| **gesamt** | 300,00 | 924,00 |

Die Einzelsubstanzen werden gemäß den Angaben in der mittleren Tabellenspalte gemischt und in an sich bekannter Weise zu einem Wirkstoffgranulat bzw. -pulver verarbeitet. Die Zusammensetzung wird anschließend zu Magentabletten verarbeitet, wobei die resultierenden Tabletten jeweils die in der rechten Tabellenspalte angegebenen Wirk-/Hilfsstoffe bzw. Gleitmittel aufweisen. Das Wirkstoffgranulat bzw. -pulver wird dabei auf einem Korsch-Exzenter verpreßt. Der Feuchtegehalt der resultierenden Tabletten beträgt etwa 3 %. Die hergestellten Magentabletten weisen hinsichtlich ihrer Form eine Breite von etwa 13 mm und eine Höhe von etwa 6 mm bei einer Härte von etwa 115 N auf. Das Gewicht der resultierenden Magentabletten beträgt etwa 924 mg/Tablette. Die Tabletten zerfallen bei Applikation im Magen innerhalb von etwa 16 Minuten.

### 2. Zusammensetzung B (erfindungsgemäß):

In einem weiteren Ansatz wird eine erfindungsgemäße Zusammensetzung hergestellt, welche die folgenden Inhaltsstoffe aufweist:

| **Zusammensetzung:** | **mg/Einheit** |
|---|---|
| Heilerde | 1.000 |
| Alginsäure | 500 |
| Natriumhydrogencarbonat | 100 |
| Additive (Verarbeitungshilfsmittel, Aromen, Süßungsmittel, Säuerungsmittel, Stabilisatoren) | ad 4.700 |
| | Gesamtgewicht 4.700 |

Die resultierende Zusammensetzung wird in Form eines Pulvers mit einem Füllgewicht von je 4.700 mg in Dosierbeutel abgefüllt.

### 3. Zusammensetzung C (erfindungsgemäß):

Es wird eine erfindungsgemäße Zusammensetzung hergestellt. Die erfindungsgemäße Zusammensetzung enthält dabei die folgenden Inhaltsstoffe:

| **Zusammensetzung:** | **mg/Einheit** |
|---|---|
| Heilerde | 500 |
| Alginsäure | 500 |
| Natriumhydrogencarbonat | 100 |
| Additive (Verarbeitungshilfsmittel, Aromen, Süßungsmittel, Stabilisatoren) | ad 4.500 |
| | Gesamtgewicht 4.500 |

Die resultierende Zusammensetzung wird in an sich bekannter Weise unter Wasserzugabe zu einer anwendungsfertigen Suspension verarbeitet, welche nachfolgend in dosierfertige Suspensionsbeutel abgefüllt wird. Geeignete Dosiereinheiten bzw. Suspensionsbeutel umfassen beispielsweise 20 bis 30 ml der erfindungsgemäßen Suspension, welche sich beispielsweise durch Zugabe geeigneter Wassermengen (z. B. 20 bis 30 g pro Dosiereinheit) herstellen läßt.

### 4. Anwendungsbeobachtungen bzw. Wirksamkeitsstudien:

Im Rahmen einer Wirksamkeitsstudie wurden insgesamt 16 Patienten, bei denen klinisch und endoskopisch eine Refluxösophagitis diagnostiziert wurde und bei denen die damit einhergehende Symptomatik schon länger als ein Jahr vor Beginn der Anwendungsbeobachtungen vorlag, zunächst in zwei Untersuchungsgruppen aus jeweils acht Patienten unterteilt. Die Untersuchungsgruppen bestanden jeweils aus vier männlichen und vier weiblichen Probanden, wobei das Durchschnittsalter in der ersten Untersuchungsgruppe (Gruppe I) 53,5 Jahre betrug, während in bezug auf die zweite Untersuchungsgruppe (Gruppe II) ein Altersdurchschnitt von 51,7 Jahren ermittelt wurde.

Bei der Gruppe I wurde eine erfindungsgemäße Zusammensetzung auf Basis der zuvor beschriebenen Zusammensetzung C - d. h. mit 500 mg Heilerde, 500 mg Alginsäure sowie 100 mg Natriumhydrogencarbonat pro Dosiereinheit - verabreicht. Die zu verabreichende Zusammensetzung C lag in Form einer in Suspensionsbeutel eingebrachten Suspension mit einem Volumen von je etwa 25 ml vor.

Die Gruppe II erhielt demgegenüber eine nichterfindungsgemäße Zusammensetzung, wobei die nichterfindungsgemäße Zusammensetzung der zuvor beschriebenen erfindungsgemäßen Zusammensetzung C mit der Ausnahme entsprach, daß anstelle von Natriumhydrogencarbonat 100 mg eines üblichen Hilfs- bzw. Füllstoffes eingesetzt wurde. Die nichterfindungsgemäße Zusammensetzung für die Gruppe II enthielt somit kein Natriumhydrogencarbonat. Die zu verabreichende nichterfindungsgemäße Zusammensetzung lag in Form einer in Suspensionsbeutel eingebrachten Suspension mit einem Volumen von jeweils etwa 25 ml vor.

Das Applikationsschema sowohl für die Gruppe I als auch für die Gruppe II war wie folgt: Die Therapie wurde über die nachfolgend genannten Zeiträume durchgeführt, wobei jeweils der Inhalt eines Suspensionsbeutels ein bis zwei Stunden nach den Mahlzeiten verabreicht wurde sowie der Inhalt eines weiteren Suspensionsbeutels täglich vor dem Nachtschlaf eingenommen wurde.

Bei beiden Patientengruppen zeigte sich bereits kurz nach Einnahme der jeweiligen Suspensionen eine Linderung des Sodbrennens, wobei jedoch bei der Vergleichsgruppe II der Effekt der Linderung des Sodbrennens weniger stark ausgeprägt war und auch nicht langanhaltend erfolgte (ca. ½ bis 1 Stunde), wohingegen bei der Probandengruppe I mit der Suspension auf Basis der erfindungsgemäßen Zusammensetzung C der Effekt bis zur nächsten Mahlzeit andauerte.

Nach bereits drei Wochen war im Fall der Gruppe I mit der erfindungsgemäßen Zusammensetzung in sämtlichen Fällen die Refluxösophagitis vollständig austherapiert, wobei die Therapie zur Verhinderung von Rezidiven noch weitere vier Wochen fortgesetzt wurde.

Demgegenüber dauerte die Behandlung der Gruppe II mit der nichterfindungsgemäßen Zusammensetzung bis zu einer gewissen Austherapierung zwei Wochen länger (somit fünf Wochen nach Therapiebeginn), wobei bei insgesamt drei Probanden auch nach dieser Zeit keine vollständige Ausheilung der Refluxösophagitis vorlag; bei diesen Patienten wurde die Therapie mit dem nichterfindungsgemäßen Präparat auf Basis von Heilerde und Alginsäure ohne Natriumhydrogencarbonat fortgeführt, wobei dann nach drei weiteren Wochen auch bei diesen Patienten eine vollständige Ausheilung der Refluxösophagitis eintrat und die Therapie nachfolgend vier weitere Wochen fortgesetzt wurde, um Rezidive zu verhindern. Auch bei den fünf Probanden der Gruppe II, bei denen ein gewisser Therapieerfolg fünf Wochen nach Therapiebeginn beobachtet wurde, wurde die Therapie zur Verhinderung von Rezidiven nach diesen fünf Wochen noch für weitere vier Wochen fortgesetzt.

Die Patienten der Gruppe I vertrugen die erfindungsgemäße Zusammensetzung sehr gut, und es traten in diesem Zusammenhang keine schwerwiegenden Nebenwirkungen auf.

Alle Diagnosen und Anamnesen wurden klinisch wie endoskopisch ermittelt und verfolgt.

Die vorliegende Patientenstudie belegt in eindrucksvoller Weise die hervorragende Wirkung und Überlegenheit der erfindungsgemäßen ternären Wirkstoffkombination aus Heilerde, Alginsäure und Natriumhydrogencarbonat, wobei die vorliegende Patientenstudie gleichermaßen die gute Verträglichkeit der erfindungsgemäßen Zusammensetzung auch bei längeren Applikationsdauern bei gleichzeitig hervorragender Langzeitwirkung belegt.

## Patentansprüche

1. Zusammensetzung, insbesondere pharmazeutische Zubereitung,
wobei die Zusammensetzung - in Kombination und in jeweils wirksamen, insbesondere pharmazeutisch wirksamen Mengen und jeweils bezogen auf die Zusammensetzung -
(a) 5 bis 80 Gew.-% Heilerde; und
(b) 1 bis 70 Gew.-% Alginsäure und/oder mindestens ein physiologisch verträgliches Alginsäuresalz;
zusammen mit
(c) 0,1 bis 50 Gew.-% mindestens einer unter physiologischen Bedingungen im Magen und/oder unter Einwirkung von Säure, insbesondere Salzsäure, gasfreisetzenden Komponente, wobei die gasfreisetzende Komponente ein Carbonat und/oder Hydrogencarbonat ist,
enthält.

2. Zusammensetzung nach Anspruch 1, wobei die Zusammensetzung außerdem einen pharmazeutischen Träger enthält und/oder wobei die Zusammensetzung in peroral verabreichbarer Applikationsform vorliegt.

3. Zusammensetzung nach Anspruch 1 oder 2, wobei die Zusammensetzung in fester oder flüssiger Dosierungsform vorliegt.

4. Zusammensetzung nach Anspruch 3, wobei die Zusammensetzung in fester Dosierungsform, insbesondere als Pulver, Granulat oder Tablette, vorzugsweise als Tablette, bevorzugt als Kautablette, vorliegt oder wobei die Zusammensetzung in flüssiger Dosierungsform, insbesondere als Suspension, vorliegt.

5. Zusammensetzung nach einem oder mehreren der vorangehenden Ansprüche, wobei (a) die Heilerde mindestens einen Bestandteil aus der Gruppe von Silikaten, Dreischichttonmineralen, insbesondere Illit, Smectid und/oder Colorit, Feldspaten, Kalzit und Dolomit enthält und/oder wobei (a) die Heilerde, insbesondere in gebundener Form, mindestens ein chemisches Element, ausgewählt aus der Gruppe von Sauerstoff, Silizium, Calcium, Aluminium, Eisen, Kalium, Magnesium, Natrium, Titan und Phosphor, enthält und/oder wobei (a) die Heilerde Spurenelemente, insbesondere ausgewählt aus der Gruppe von Kupfer, Mangan, Nickel, Selen und Zink, enthält.

6. Zusammensetzung nach einem oder mehreren der vorangehenden Ansprüche, wobei die Zusammensetzung die Wirkstoffkomponenten (a) und (b) in einem massenbezogenen Verhältnis von (a) / (b) im Bereich von 0,1 - 10 / 0,1 - 10, insbesondere 0,5 - 5 / 0,5 - 5, vorzugsweise 0,75 - 3 / 0,75 - 3, bevorzugt 1 - 2 / 1 - 2, enthält.

7. Zusammensetzung nach einem oder mehreren der vorangehenden Ansprüche, wobei die Zusammensetzung (a) die Heilerde pro Dosiereinheit, insbesondere je Tablette oder je Dosierbeutel oder je Suspensionsbeutel, in Mengen von 10 bis 5.000 mg, insbesondere 50 bis 3.000 mg, vorzugsweise 100 bis 2.000 mg, besonders bevorzugt 200 bis 1.500 mg, ganz besonders bevorzugt 400 bis 1.000 mg, enthält und/oder wobei die Zusammensetzung (b) die Alginsäure und/oder das Alginsäuresalz pro Dosiereinheit, insbesondere je Tablette oder je Dosierbeutel oder je Suspensionsbeutel, in Mengen von insgesamt 10 bis 1.500 mg, insbesondere 50 bis 1.250 mg, vorzugsweise 75 bis 1.000 mg, besonders bevorzugt 100 bis 800 mg, ganz besonders bevorzugt 150 bis 600 mg, enthält.

8. Zusammensetzung nach einem oder mehreren der vorangehenden Ansprüche, wobei die Zusammensetzung (b) die Alginsäure und/oder das Alginsäuresalz pro Dosiereinheit, insbesondere je Tablette oder je Dosierbeutel oder je Suspensionsbeutel, in Mengen von insgesamt 10 bis 1.500 mg, insbesondere 50 bis 1.250 mg, vorzugsweise 75 bis 1.000 mg, besonders bevorzugt 100 bis 800 mg, ganz besonders bevorzugt 150 bis 600 mg, enthält.

9. Zusammensetzung nach einem oder mehreren der vorangehenden Ansprüche, wobei (c) die unter physiologischen Bedingungen im Magen und/oder unter Einwirkung von Säure, insbesondere Salzsäure, gasfreisetzende Komponente ein Erdalkalicarbonat und/oder Erdalkalihydrogencarbonat, insbesondere des Magnesiums und/oder des Calciums, ist und/oder wobei (c) die unter physiologischen Bedingungen im Magen und/oder unter Einwirkung von Säure, insbesondere Salzsäure, gasfreisetzende Komponente ein Alkalicarbonat und/oder Alkalihydrogencarbonat, insbesondere des Kaliums und/oder des Natriums, vorzugsweise des Natriums, ist und/oder wobei (c) die unter physiologischen Bedingungen im Magen und/oder unter Einwirkung von Säure, insbesondere Salzsäure, gasfreisetzende Komponente Natriumhydrogencarbonat ist.

10. Zusammensetzung nach einem oder mehreren der vorangehenden Ansprüche, wobei die Zusammensetzung die Wirkstoffkomponenten (a), (b) und (c) in einem massenbezogenen Verhältnis von (a)/(b)/(c) im Bereich von 0,01 - 50 / 0,01 - 20 / 0,1 - 10, insbesondere 0,1 - 20 / 0,1 - 10 / 0,1 - 5, vorzugsweise 0,1-15 / 0,5 - 8 / 0,1 - 3, bevorzugt 5 - 10 / 5 / 1, enthält.

11. Zusammensetzung, insbesondere pharmazeutische Zubereitung
, nach einem oder mehreren der vorangehenden Ansprüche, wobei die Zusammensetzung - in Kombination und in jeweils wirksamen, insbesondere pharmazeutisch wirksamen Mengen und jeweils bezogen auf die Zusammensetzung -
(a) Heilerde, insbesondere in Mengen von 5 bis 80 Gew.-%, insbesondere 10 bis 70 Gew.-%, vorzugsweise 15 bis 50 Gew.-%, besonders bevorzugt 15 bis 40 Gew.-%, ganz besonders bevorzugt 20 bis 30 Gew.-%, und/oder insbesondere wobei (a) die Heilerde mindestens einen Bestandteil aus der Gruppe von Silikaten, Dreischichttonmineralen, insbesondere Illit, Smectid und/oder Colorit, Feldspaten, Kalzit und Dolomit enthält;
(b) Alginsäure und/oder mindestens ein physiologisch verträgliches Alginsäuresalz, insbesondere in Mengen von insgesamt 1 bis 70 Gew.-%, insbesondere 5 bis 60 Gew.-%, vorzugsweise 10 bis 55 Gew.-%, besonders bevorzugt 15 bis 50 Gew.-%, ganz besonders bevorzugt 20 bis 40 Gew.-%;
zusammen mit
(c) mindestens einer unter physiologischen Bedingungen im Magen und/oder unter Einwirkung von Säure, insbesondere Salzsäure, gasfreisetzenden Komponente, insbesondere wobei die Komponente (c) ein Erdalkalicarbonat und/oder Erdalkalihydrogencarbonat, insbesondere des Magnesiums und/oder des Calciums, ist und/oder insbesondere wobei die Komponente (c) ein Alkalicarbonat und/oder Alkalihydrogencarbonat, insbesondere des Kaliums und/oder des Natrium, vorzugsweise des Natriums, ist, und/oder insbesondere wobei die Komponente (c) Natriumhydrogencarbonat ist, und/oder insbesondere wobei die Zusammensetzung die Komponente (c) in Mengen von insgesamt 0,1 bis 50 Gew.-%, insbesondere 0,5 bis 30 Gew.-%, vorzugsweise 1 bis 20 Gew.-%, besonders bevorzugt 1,5 bis 10 Gew.-%, ganz besonders bevorzugt 2 bis 10 Gew.-%, enthält;
enthält.

12. Zusammensetzung, insbesondere pharmazeutische Zubereitung,
nach einem oder mehreren der vorangehenden Ansprüche, wobei die Zusammensetzung - in Kombination und in jeweils wirksamen, insbesondere pharmazeutisch wirksamen Mengen -
(a) Heilerde, insbesondere wobei (a) die Heilerde mindestens einen Bestandteil aus der Gruppe von Silikaten, Dreischichttonmineralen, insbesondere Illit, Smectid und/oder Colorit, Feldspaten, Kalzit und Dolomit enthält, insbesondere wobei die Zusammensetzung die (a) Heilerde pro Dosiereinheit, insbesondere je Tablette oder je Dosierbeutel oder je Suspensionsbeutel, in Mengen von 10 bis 5.000 mg, insbesondere 50 bis 3.000 mg, vorzugsweise 100 bis 2.000 mg, besonders bevorzugt 200 bis 1.500 mg, ganz besonders bevorzugt 400 bis 1.000 mg, enthält;
(b) Alginsäure und/oder mindestens ein physiologisch verträgliches Alginsäuresalz, insbesondere wobei die Zusammensetzung (b) die Alginsäure und/oder das Alginsäuresalz pro Dosiereinheit, insbesondere je Tablette oder je Dosierbeutel oder je Suspensionsbeutel, in Mengen von insgesamt 10 bis 1.500 mg, insbesondere 50 bis 1.250 mg, vorzugsweise 75 bis 1.000 mg, besonders bevorzugt 100 bis 800 mg, ganz besonders bevorzugt 150 bis 600 mg, enthält;
zusammen mit
(c) mindestens einer unter physiologischen Bedingungen im Magen und/oder unter Einwirkung von Säure, insbesondere Salzsäure, gasfreisetzenden Komponente, insbesondere wobei die Komponente (c) ein Erdalkalicarbonat und/oder Erdalkalihydrogencarbonat, insbesondere des Magnesiums und/oder des Calciums, ist und/oder insbesondere wobei die Komponente (c) ein Alkalicarbonat und/oder Alkalihydrogencarbonat, insbesondere des Kaliums und/oder des Natriums, vorzugsweise des Natriums, ist, und/oder insbesondere wobei die Komponente (c) Natriumhydrogencarbonat ist, und/oder insbesondere wobei die Zusammensetzung die Komponente (c), insbesondere je Tablette oder je Dosierbeutel oder je Suspensionsbeutel, in Mengen von 1 bis 500 mg, insbesondere 5 bis 300 mg, vorzugsweise 10 bis 200 mg, besonders bevorzugt 20 bis 150 mg, ganz besonders bevorzugt 30 bis 130 mg, enthält;
enthält.

13. Zusammensetzung, insbesondere pharmazeutische Zubereitung,
nach einem oder mehreren der vorangehenden Ansprüche, wobei die Zusammensetzung - in Kombination und in jeweils wirksamen, insbesondere pharmazeutisch wirksamen Mengen pro Dosiereinheit, insbesondere je Tablette oder je Dosierbeutel oder je Suspensionsbeutel -
(a) Heilerde, insbesondere in Mengen von 400 bis 1.000 mg;
(b) Alginsäure und/oder mindestens ein physiologisch verträgliches Alginsäuresalz, insbesondere in Mengen von insgesamt 150 bis 600 mg;
zusammen mit
(c) Natriumhydrogencarbonat, insbesondere in Mengen von 30 bis 130 mg; enthält.

14. Verwendung einer Zusammensetzung nach den vorangehenden Ansprüchen zur Herstellung eines Arzneimittels zur Verwendung als Antazidum.

15. Verwendung einer Zusammensetzung nach den vorangehenden Ansprüchen zur Herstellung eines
Arzneimittels zur prophylaktischen und/oder kurativen, insbesondere symptomatischen prophylaktischen und/oder kurativen Behandlung von Hyperazidität und damit in Zusammenhang stehenden Folgeerscheinungen und Erkrankungen oder von Sodbrennen, Ösophagitis, insbesondere Refluxösophagitis, dyspeptischen Beschwerden, Reizmagen, Gastritis und Ulcuserkrankungen eingesetzt wird.

## Claims

1. A composition, in particular a pharmaceutical preparation, wherein the composition comprises - in combination, and each at effective, in particularly pharmaceutically effective amounts, and each based on the composition -
(a) 5 to 80% by weight healing earth; and
(b) 1 to 70% by weight alginic acid, and/or at least one physiologically
acceptable alginic acid salt;
together with
(c) 0.1 to 50% by weight of at least one component releasing gas under physiological conditions within the stomach, and/or under the influence of acid, in particular hydrochloric acid, wherein the component releasing gas is a carbonate, and/or hydrogen carbonate.

2. The composition according to claim 1, wherein the composition further comprises a pharmaceutical carrier, and/or wherein the composition is present in an application form that may be administered perorally.

3. The composition according to claims 1 or 2, wherein the composition is present in solid or liquid dosage form.

4. The composition according to claim 3, wherein the composition is present in solid dosage form, in particular as a powder, granulate, or tablet, preferably as a tablet, preferably as a chewable tablet, or wherein the composition is present in a liquid dosage form, in particular as a suspension.

5. The composition according to one or more of the previous claims, wherein (a) the healing earth comprises at least one component from the group of silicates, three-layer clay minerals, in particular illite, smectide, and/or colorite, feldspars, calcite, and dolomite, and/or wherein (a) the healing earth, in particular in bound form, comprises at least one chemical element, selected from the group of oxygen, silicon, calcium, aluminum, iron, potassium, magnesium, sodium, titanium, and phosphor, and/or wherein (a) the healing earth comprises trace elements, in particular selected from the group of copper, manganese, nickel, selenium and zinc.

6. The composition according to one or more of the previous claims, wherein the composition comprises the active components (a) and (b) in a ration based on mass of (a)/(b) in the range of 0.1-10/0.1-10, in particular 0.5/5/0.5-5, preferably 0.75-3/0.75-3, preferably 1-2/1-2.

7. The composition according to one or more of the previous claims, wherein the composition comprises (a) the healing earth per dosage unit, in particular per tablet, or per dosage bag, or per suspension bag, in amounts from 10 to 5,000 mg, in particular 50 to 3,000 mg, preferably 100 to 2,000 mg, particularly preferred 200 to 1,500 mg, most particularly preferred 400 to 1,000 mg, and/or wherein the composition (b) comprises the alginic acid and/or the alginic acid salt per dosage unit, in particular per tablet, or per dosage bag, or per suspension bag, in amounts of a total of 10 to 1,500 mg, in particular 50 to 1,250 mg, preferably 75 to 1,000 mg, particularly preferred 100 to 800 mg, most preferred 150 to 600 mg.

8. The composition according to one or more of the previous claims, wherein the composition (b) comprises the alginic acid and/or the alginic acid salt per dosage unit, in particular per tablet, or per dosage bag, or per suspension bag, in amounts of a total of 10 to 1,500 mg, in particular 50 to 1,250 mg, preferably 75 to 1,000 mg, particularly preferred 100 to 800 mg, most preferred 150 to 600 mg.

9. The composition according to one or more of the previous claims, wherein (c) the component releasing gas under physiological conditions within the stomach, and/or under the influence of acid, in particular hydrochloric acid, is an alkaline earth carbonate, and/or an alkaline earth hydrogen carbonate, in particular of magnesium and/or of calcium, and/or wherein (c) the component releasing gas under physiological conditions within the stomach, and/or under the influence of acid, in particular hydrochloric acid, is an alkaline carbonate, and/or an alkaline hydrogen carbonate, in particular of potassium and/or of sodium, preferably of sodium, and/or wherein (c) the component releasing gas under physiological conditions within the stomach, and/or under the influence of acid, in particular hydrochloric acid, is sodium hydrogen carbonate.

10. The composition according to one or more of the previous claims, wherein the composition comprises the active components (a), (b), and (c) in a ratio based on mass of (a)/(b)/(c) in the range of 0.01-50/0.01-20/0.1-10, in particular 0.1-20/0.1-10/0.1-5, preferably 0.1-15/0.5-8/0.1-3, preferably 5-10/5/1.

11. The composition, in particular the pharmaceutical preparation, according to one or more of the previous claims, wherein the composition comprises - in combination of each effective, in particular pharmaceutically effective amounts, and each based on the composition -
(a) healing earth, in particular in amounts from 5 to 80% by weight, in particular 10 to 70% by weight, preferably 15 to 50% by weight, particularly preferred 15 to 40% by weight, most preferred 20 to 30% by weight, and/or in particular wherein (a) the healing earth comprises at least one component from the group of silicates, three-layer clay minerals, in particular illite, smectide, and/or colorite, feldspars, calcite, and dolomite;
(b) alginic acid, and/or at least one physiologically acceptable alginic acid salt, in particular in amounts of a total of 1 to 70% by weight, in particular 5 to 60% by weight, preferably 10 to 55% by weight, particularly preferred 15 to 50% by weight, most preferred 20 to 40% by weight;
together with
(c) at least one component releasing gas under physiological conditions within the stomach, and/or under the influence of acid, in particular hydrochloric acid, in particular wherein the component (c) is an alkaline earth carbonate and/or an alkaline earth hydrogen carbonate, in particular of magnesium and/or of calcium, and/or in particular wherein the component (c) is an alkaline carbonate and/or an alkaline hydrogen carbonate, in particular of potassium and/or of sodium, preferably of sodium, and/or in particular wherein the component (c) is sodium hydrogen carbonate, and/or in particular wherein the composition comprises the component (c) in amounts of a total of 0.1 to 50% by weight, in particular 0.5 to 30% by weight, preferably 1 to 20% by weight, particularly preferred 1.5 to 10% by weight, most preferred 2 to 10% by weight.

12. The composition, in particular the pharmaceutical preparation, according to one or more of the previous claims, wherein the composition comprises - in combination, and each in effective, in particular pharmaceutically effective, amounts -
(a) healing earth, in particular wherein (a) the healing earth comprises at least one component from the group of silicates, three-layer clay minerals, in particular illite, smectide, and/or colorite, feldspars, calcite, and dolomite, in particular wherein the composition comprises the (a) healing earth per dosage unit, in particular per tablet, or per dosage bag, or per suspension bag, in amounts from 10 to 5,000 mg, in particular 50 to 3,000 mg, preferably 100 to 2,000 mg, particularly preferred 200 to 1,500 mg, most particularly preferred 400 to 1,000 mg;
(b) alginic acid, and/or at least one physiologically acceptable alginic acid, in particular wherein the composition (b) comprises the alginic acid and/or the alginic acid salt per dosage unit, in particular per tablet, or per dosage bag, or per suspension bag, in amounts of a total of 10 to 1,500 mg, in particular 50 to 1,250 mg, preferably 75 to 1,000 mg, particularly preferred 100 to 800 mg, most preferred 150 to 600 mg;
together with
(c) at least one component releasing gas under physiological conditions within the stomach, and/or under the influence of acid, in particular hydrochloric acid, in particular wherein the component (c) is an alkaline earth carbonate and/or an alkaline earth hydrogen carbonate, in particular of magnesium and/or of calcium, and/or in particular wherein the component (c) is an alkaline carbonate and/or an alkaline hydrogen carbonate, in particular of potassium and/or of sodium, preferably of sodium, and/or in particular wherein the component (c) is sodium hydrogen carbonate, and/or in particular wherein the composition comprises the component (c), in particular per table, or per dosage bag, or per suspension bag, in amounts from 1 to 500 mg, in particular 5 to 300 mg, preferably 10 to 200 mg, particularly preferred 20 to 150 mg, most preferred 30 to 130 mg.

13. The composition, in particular the pharmaceutical preparation, according to one or more of the previous claims, wherein the composition comprises - in combination, and each in effective, in particular pharmaceutically effective amounts per dosage unit, in particular per table, or per dosage bag, or per suspension bag -
(a) healing earth, in particular in amounts of 400 to 1,000 mg;
(b) alginic acid, and/or at least one physiologically acceptable alginic acid
salt, in particular in amounts of a total of 150 to 600 mg;
together with
(c) sodium hydrogen carbonate, in particular in amounts of 30 to 130 mg.

14. A use of a composition according to any of the previous claims for the production of pharmaceuticals for the use as an antacid.

15. The use of a composition according to any of the previous claims for the production of a pharmaceutical for the prophylactic and/or curative, in particular symptomatic prophylactic and/or curative treatment of hyperacidity, and any aftereffects and diseases associated with the same, or of heart burn, esophagitis, in particular reflux esophagitis, dyspeptic disorders, irritable bowel syndrome, gastritis, and ulcerous diseases.

## Revendications

1. Composition, en particulier préparation pharmaceutique, la composition contenant - en combinaison et chacun en des quantités actives, en particulier pharmaceutiquement actives, chaque pourcentage étant rapporté à la composition -
(a) 5 à 80 % en poids d'argile médicinale; et
(b) 1 à 70 % en poids d'acide alginique et/ou d'un sel physiologiquement
acceptable de l'acide alginique;
en même temps que
(c) 0,1 à 50 % en poids d'au moins un composant libérant un gaz dans des conditions physiologiques dans l'estomac et/ou sous l'action d'un acide, en particulier l'acide chlorhydrique, le composant libérant un gaz étant un carbonate et/ou un hydrogénocarbonate.

2. Composition selon la revendication 1, la composition contenant en outre un support pharmaceutique, et/ou la composition se présentant sous une forme galénique pouvant être administrée par voie perorale.

3. Composition selon la revendication 1 ou 2, la composition se présentant sous une forme posologique solide ou liquide.

4. Composition selon la revendication 3, la composition se présentant sous une forme posologique solide, en particulier d'une poudre, d'un granule ou d'un comprimé, de préférence sous forme d'un comprimé, d'une manière préférée sous forme d'un comprimé à mâcher, la composition se présentant sous une forme posologique liquide, en particulier d'une suspension.

5. Composition selon l'une ou plusieurs des revendications précédentes, dans laquelle (a) l'argile médicinale contient au moins un constituant du groupe consistant en les silicates, les minéraux argileux à trois couches réticulaires, en particulier l'illite, la smectite et/ou le chlorite, les feldspaths, la calcite et la dolomite, et/ou dans laquelle (a) l'arigle médicinale, en particulier sous forme liée, contient au moins un élément chimique choisi dans le groupe consistant en l'oxygène, le silicium, le calcium, l'aluminium, le fer, le potassium, le magnésium, le sodium, le titane et le phosphore, et/ou dans laquelle (a) l'argile médicinale contient des oligoéléments choisis en particulier dans le groupe consistant en le cuivre, le manganèse, le nickel, le sélénium et le zinc.

6. Composition selon l'une ou plusieurs des revendications précédentes, la composition contenant les composants actifs (a) et (b) selon un rapport en masse (a)/(b) compris dans la plage de 0,1-10 / 0,1-10, en particulier de 0,5-5 / 0,5-5, de préférence de 0,75-3 / 0,75-3, d'une manière préférée de 1-2/1-2.

7. Composition selon l'une ou plusieurs des revendications précédentes, la composition contenant (a) l'argile médicinale, par unité posologique, en particulier par comprimé ou par sachet doseur ou par sachet de suspension, en des quantités de 10 à 5000 mg, en particulier de 50 à 3000 mg, de préférence de 100 à 2000 mg, d'une manière particulièrement préférée de 200 à 1500 mg, d'une manière tout particulièrement préférée de 400 à 1000 mg, et/ou la composition contenant (b) l'acide alginique et/ou le sel de l'acide alginique, par unité posologique, en particulier par comprimé ou par sachet doseur ou par sachet de suspension, en des quantités globales de 10 à 1500 mg, en particulier de 50 à 1250 mg, de préférence de 75 à 1000 mg, d'une manière particulièrement préférée de 100 à 800 mg, d'une manière tout particulièrement préférée de 150 à 600 mg.

8. Composition selon l'une ou plusieurs des revendications précédentes, la composition contenant (b) l'acide alginique et/ou le sel de l'acide alginique, par unité posologique, en particulier par comprimé ou par sachet doseur ou par sachet de suspension, en des quantités globales de 10 à 1500 mg, en particulier de 50 à 1250 mg, de préférence de 75 à 1000 mg, d'une manière particulièrement préférée de 100 à 800 mg, d'une manière tout particulièrement préférée de 150 à 600 mg.

9. Composition selon l'une ou plusieurs des revendications précédentes, dans laquelle (c) le composant libérant un gaz dans des conditions physiologiques dans l'estomac et/ou sous l'action d'un acide, en particulier l'acide chlorhydrique, est le carbonate d'un métal alcalino-terreux et/ou l'hydrogénocarbonate d'un métal alcalino-terreux, en particulier de magnésium et/ou de calcium, et/ou dans laquelle (c) le composant libérant un gaz dans des conditions physiologiques dans l'estomac et/ou sous l'action d'un acide, en particulier l'acide chlorhydrique, est le carbonate d'un métal alcalin et/ou l'hydrogénocarbonate d'un métal alcalin, en particulier de potassium et/ou de sodium, de préférence de sodium, et/ou dans laquelle (c) le composant libérant un gaz dans des conditions physiologiques dans l'estomac et/ou sous l'action d'un acide, en particulier l'acide chlorhydrique, est l'hydrogénocarbonate de sodium.

10. Composition selon l'une ou plusieurs des revendications précédentes, la composition contenant les composants actifs (a), (b) et (c) selon une proportion en masse (a)/(b)/(c) comprise dans la plage de 0,01-50 / 0,01-20 / 0,1 - 10, en particulier de 0,1-20 / 0,1-10 / 0,1-5, de préférence de 0,1-15 / 0,5-8 / 0,1-3, de préférence de 5-10 / 5 / 1.

11. Composition, en particulier préparation pharmaceutique selon l'une ou plusieurs des revendications précédentes, la composition contenant - en combinaison, et chacun en des quantités actives, en particulier pharmaceutiquement actives, chaque pourcentage étant rapporté à la composition -
(a) une argile médicinale, en particulier en des quantités de 5 à 80 % en poids, en particulier de 10 à 70 % en poids, de préférence de 15 à 50 % en poids, d'une manière particulièrement préférée de 15 à 40 % en poids, d'une manière tout particulièrement préférée de 20 à 30 % en poids et/ou en particulier dans laquelle (a) l'argile médicinale contient au moins un constituant du groupe consistant en les silicates, les minéraux argileux à trois couches réticulaires, en particulier l'illite, la smectite et/ou la chlorite, les feldspaths, la calcite et la dolomite;
(b) de l'acide alginique et/ou au moins un sel de l'acide alginique physiologiquement acceptable, en particulier en des quantités globales de 1 à 70 % en poids, en particulier de 5 à 60 % en poids, de préférence de 10 à 55 % en poids, d'une manière particulièrement préférée de 15 à 50 % en poids, d'une manière tout particulièrement préférée de 20 à 40 % en poids;
en même temps que
(c) au moins un composant libérant un gaz dans des conditions physiologiques dans l'estomac et/ou sous l'action d'un acide, en particulier l'acide chlorhydrique, en particulier dans laquelle le composant (c) est le carbonate d'un métal alcalin et/ou l'hydrogénocarbonate d'un métal alcalin, en particulier de magnésium et/ou de calcium, et/ou en particulier dans laquelle le composant (c) est le carbonate d'un métal alcalin et/ou l'hydrogénocarbonate d'un métal alcalin, en particulier de potassium et/ou de sodium, de préférence de sodium, et/ou en particulier dans laquelle le composant (c) est l'hydrogénocarbonate de sodium, et/ou en particulier dans laquelle la composition contient le composant (c) en des quantités globales de 0,1 à 50 % en poids, en particulier de 0,5 à 30 % en poids, de préférence de 1 à 20 % en poids, d'une manière particulièrement préférée de 1,5 à 10 % en poids, d'une manière tout particulièrement préférée de 2 à 10 % en poids.

12. Composition, en particulier préparation pharmaceutique selon l'une ou plusieurs des revendications précédentes, la combinaison contenant - en combinaison, et chacun dans des quantités actives, en particulier pharmaceutiquement actives -
(a) de l'argile médicinale, en particulier dans laquelle (a) l'argile médicinale contient au moins un constituant du groupe consistant en les silicates, les minéraux argileux à trois couches réticulaires, en particulier l'illite, la smectite et/ou la chlorite, les feldspaths, la calcite et la dolomite, la composition contenant en particulier l'argile médicinale (a) par unité posologique, en particulier par comprimé ou par sachet doseur ou par sachet de suspension, en des quantités de 10 à 5000 mg, en particulier de 50 à 3000 mg, de préférence de 100 à 2000 mg, d'une manière particulièrement préférée de 200 à 1500 mg, d'une manière tout particulièrement préférée de 400 à 1000 mg;
(b) de l'acide alginique et/ou un sel de l'acide alginique physiologiquement acceptable, en particulier la composition contenant (b) l'acide alginique et/ou le sel de l'acide alginique, par unité posologique, en particulier par comprimé ou par sachet doseur ou par sachet de suspension, en des quantités globales de 10 à 1500 mg, en particulier de 50 à 1250 mg, de préférence de 75 à 1000 mg, d'une manière particulièrement préférée de 100 à 800 mg, d'une manière tout particulièrement préférée de 150 à 600 mg;
en même temps que
(c) au moins un composant libérant un gaz dans des conditions physiologiques dans l'estomac et/ou sous l'action d'un acide, en particulier l'acide chlorhydrique, dans laquelle le composant (c) est le carbonate d'un métal alcalino-terreux et/ou l'hydrogénocarbonate d'un métal alcalino-terreux, en particulier de magnésium et/ou de calcium, et/ou en particulier dans laquelle le composant (c) est le carbonate d'un métal alcalin et/ou l'hydrogénocarbonate d'un métal alcalin, en particulier de potassium et/ou de sodium, de préférence de sodium, et/ou en particulier dans laquelle le composant (c) est l'hydrogénocarbonate de sodium, et/ou en particulier dans laquelle la composition contient le composant (c), en particulier par comprimé ou par sachet doseur ou par sachet de suspension, en des quantités de 1 à 500 mg, en particulier de 5 à 300 mg, de préférence de 10 à 200 mg, d'une manière particulièrement préférée de 20 à 150 mg, d'une manière tout particulièrement préférée de 30 à 130 mg.

13. Composition, en particulier préparation pharmaceutique selon l'une ou plusieurs des revendications précédentes, la composition contenant - en combinaison, et chacun en des quantités actives, en particulier pharmaceutiquement actives, par unité posologique, en particulier par comprimé ou par sachet doseur ou par sachet de suspension -
(a) une argile médicinale, en particulier en des quantités de 400 à 1000 mg;
(b) de l'acide alginique et/ou au moins un sel de l'acide alginique physiologiquement acceptable, en particulier en des quantités globales de 150 à 600 mg;
en même temps que
(c) de l'hydrogénocarbonate de sodium, en particulier en des quantités de 30 à 130 mg.

14. Utilisation d'une composition selon les revendications précédentes pour fabriquer un médicament destiné à être utilisé en tant qu'antiacide.

15. Utilisation d'une composition selon l'une des revendications précédentes pour la fabrication d'un médicament destiné au traitement prophylactique et/ou curatif, en particulier symptomatique prophylactique et/ou curatif de l'hyperacidité et des séquelles et maladies qui y sont associées, ou du pyrosis, de l'oesophagite, en particulier de l'oesophagite peptique, des troubles dyspeptiques, de l'estomac irritable, de la gastrite et des maladies ulcéreuses.
